# EUROPÄISCHE PATENTSCHRIFT

(11) **EP 4 117 749 B1**
(45) Veröffentlichungstag und Bekanntmachung des Hinweises auf die Patenterteilung: **07.05.2025**
(21) Anmeldenummer: 21707925.0
(22) Anmeldetag: 22.02.2021
(51) Int. Cl.: A61M 1/16

(54) **KONZENTRATBEHÄLTERADAPTER UND KONNEKTORGEHÄUSE ZUR ANBINDUNG AN EINE BLUTBEHANDLUNGSVORRICHTUNG**
CONCENTRATE CONTAINER ADAPTER AND CONNECTOR HOUSING FOR CONNECTION TO A BLOOD TREATMENT DEVICE
ADAPTATEUR DE CONTENEUR DE CONCENTRÉ ET BOÎTIER DE CONNECTEUR POUR LA CONNEXION À UN DISPOSITIF DE TRAITEMENT SANGUIN

(30) Priorität: 11.03.2020 DE 102020106595
(43) Veröffentlichungstag der Anmeldung: 18.01.2023
(73) Patentinhaber: Fresenius Medical Care Deutschland GmbH, 61352 Bad Homburg (DE)
(72) Erfinder: BREHM, Winfried, 97461 Hofheim (DE); STERZER, Rafael, 97422 Schweinfurt (DE); SEIT, Paul, 97421 Schweinfurt (DE); PLADT, Johannes, 97424 Schweinfurt (DE); BOND, Oliver, 97520 Heidenfeld (DE)
(74) Vertreter: Wagner, Andrea
(86) Internationale Anmeldenummer: PCT/EP2021/054283
(87) Internationale Veröffentlichungsnummer: WO 2021/180452

(56) Entgegenhaltungen:
- EP-A1- 1 344 550
- EP-A1- 2 808 042
- WO-A1-2010/106091
- WO-A2-2013/135389
- DE-A1- 102014 015 858

## Beschreibung

### Technisches Gebiet

Die vorliegende Erfindung betrifft einen Konzentratbehälteradapter für eine Blutbehandlungsvorrichtung, insbesondere eine Dialysemaschine, sowie ein Konnektorgehäuse zur Anbindung an eine Blutbehandlungsvorrichtung, vorzugsweise eine Dialysemaschine.

### Hintergrund

Unter dem Begriff der Blutbehandlungsvorrichtung ist unter anderem eine Dialysemaschine zu verstehen. Dialysemaschinen finden häufig in Dialysezentren zur Behandlung einer chronischen Nierenerkrankung Anwendung.

Während der Dialyse strömt durch die Blutkammer des Dialysators kontinuierlich Blut des Patienten, während durch die Dialysierflüssigkeitskammer fortlaufend Dialysierflüssigkeit fließt. Zur Herstellung von Dialysierflüssigkeit können vorgefertigte Dialysierflüssigkeitskonzentrate verwendet werden, die in den Dialysevorrichtungen mit Wasser verdünnt werden. In Dialysezentren werden Dialysierflüssigkeitskonzentrate entweder als vorgefertigtes Produkt in Kanistern, Beuteln oder Kartuschen zur Verfügung gestellt oder über ein Ringleitungssystem aus einem zentralen Tank bereitgestellt.

Zentral zur Verfügung gestellte Dialysierflüssigkeitskonzentrate sind für den Anwender einfach zu handhaben, sie haben aber den Nachteil, dass die Dialysierflüssigkeit nicht individuell auf die Bedürfnisse des Patienten abgestimmt werden kann. Dezentral bereitgestellte Konzentrate erlauben zwar eine individuelle Anpassung der Dialysierflüssigkeit an den Patienten, sie müssen aber für jede einzelne Dialysebehandlung an die Dialysevorrichtung gebracht werden.

In der Dialyse finden für die Herstellung eines flüssigen Dialysekonzentrats Beutel oder Kartuschen Verwendung, die mit einem pulverförmigen Dialysierflüssigkeitskonzentrat befüllt sind. Die Konzentratbeutel oder -kartuschen enthalten eine Menge an pulverförmigen Dialysierflüssigkeitskonzentrat, die für eine einzige Dialysebehandlung ausreichend ist. Die Beutel oder Kartuschen sind mit Bikarbonat befüllt. Handelsübliche Bikarbonatbeutel enthalten 650 bis 950 g Natriumbicarbonat. Aus dem pulverförmigen Bikarbonat-Konzentrat wird zunächst ein flüssiges Bikarbonat-Konzentrat hergestellt. Für die Herstellung der Dialysierflüssigkeit ist ein weiteres Säurekonzentrat erforderlich, das in einem Kanister oder mit einer zentralen Versorgung bereitgestellt wird. Bikarbonat-Konzentrat und Säurekonzentrat werden dann mit Wasser zu der fertigen Dialysierflüssigkeit gemischt.

Die Dialysemaschine weist daher ferner einen Wasseranschluss auf, über welchen Reinwasser zugeführt wird. In der Dialysemaschine erfolgt anschließend die gewünschte Mischung von Reinwasser mit dem entsprechenden Konzentrat.

Die Bereitstellung des flüssigen Konzentrat über Konzentratbehälter bringt verschiedene Herausforderungen mit sich, da die Konzentratbehälter schwer und damit für das Pflegepersonal aufwendig in der Handhabung sind. Zudem müssen zur Bereitstellung des Konzentrats Ansaugstäbe der Dialysemaschine in die Konzentratbehälter eingebracht werden, welche nach einer Behandlung zur Reinigung wieder in die Dialysemaschine gesteckt werden. Diese Handhabung der Konzentratbehälter ist damit zeitintensiv und kann zu einer Verlängerung der Pausen zwischen den einzelnen Behandlungen führen.

Alternativ kann das Konzentrat auch über eine Zentralversorgung und einen entsprechenden Zentralversorgungsanschluss an der Dialysemaschine zugeführt werden. Dies setzt aber eine entsprechende Ausstattung der Klinik voraus, und kann bezüglich der Flexibilität des zur Verfügung stehenden Konzentrats und dem Wartungsaufwand der hierfür betriebenen Leitungen Nachteile mit sich bringen.

Aus der EP 1 344 550 A1 ist ein Konnektor zur Verbindung eines Konzentratbeutels, welcher mit Trockenkonzentrat gefüllt ist und an einer Dialysemaschine eingehängt werden kann, bekannt. Auch bei dieser Art der Konzentratversorgung sind bestimmte Schritte zwischen den Behandlungen notwendig. So muss ein am Konzentratbeutel angebrachter Kontaminationsschutz manuell entfernt, der Konzentratbeutel in die entsprechende Aufnahme an der Dialysemaschine eingesetzt, das heißt konnektiert werden, und schließlich mit dem Dialysatkreislauf verbunden werden.

Nach einer Behandlung muss der Konzentratbeutel von dem Dialysatkreislauf dekonnektiert und entfernt werden. Erst nach der Entfernung kann ein Reinigungsprogramm an der Dialysemaschine gestartet werden. Auch die Oberflächenreinigung der Dialysemaschine kann erst nach dem Entfernen des Konzentratbeutels von dem Pflegepersonal durchgeführt werden.

Neben hohen Sicherheitsanforderungen spielt bei der Dialysebehandlung, insbesondere in Dialysezentren, der zeitliche Aspekt eine besondere Rolle.

Da ausreichend Zeit für die tatsächliche Behandlung zur Verfügung stehen soll, ist insbesondere die Zeit zwischen den Behandlungen einer ständigen Zeitoptimierung unterworfen. Wie oben beschrieben, nimmt zwischen den Behandlungen neben der Vorbereitung des Patienten, auch die Vorbereitung der Dialysemaschine für die nächste Behandlung, durch Schritte, wie der Bereitstellung der benötigten Konzentratbehälter oder der Desinfizierung der Dialysemaschine, Zeit in Anspruch.

Der vorliegenden Anmeldung liegt damit die Aufgabe zu Grunde eine Reinigungsvorrichtung für eine Blutbehandlungsvorrichtung bereit zu stellen, die eine verbesserte Handhabung und/oder eine Zeiteinsparung für den Nutzer erlaubt. Insbesondere soll die zur Vorbereitung der Dialysemaschine benötigte Zeit sowie die hierfür benötigten Handhabungsschritte reduziert werden können und gleichzeitig die Sicherheit des Patienten gewährleistet werden.

### Zusammenfassung der Erfindung

Die der Erfindung zugrunde liegende Aufgabe wird durch einen Konzentratbehälteradapter für eine Blutbehandlungsvorrichtung gemäß Anspruch 1 sowie ein Konnektorgehäuse zur Anbindung an eine Blutbehandlungsvorrichtung nach Anspruch 6 gelöst. Vorteilhafte Weiterentwicklungen und Ausführungsformen sind Gegenstand der abhängigen Ansprüche.

Der erfindungsgemäße Konzentratbehälteradapter zur Verwendung bei einer Blutbehandlungsvorrichtung, insbesondere einer Dialysemaschine, weist einen Befestigungskörper auf, an welchem ein Konzentratbeutel befestigbar ist. Der Befestigungskörper kann eine Befüllöffnung und zwei seitlich zur Befüllöffnung angeordnete Verbindungsöffnungen aufweisen. Weiter kann der Konzentratbehälteradapter einen Gehäuseaufnahmebereich am Befestigungskörper aufweisen, der derart eingerichtet ist, dass mittels des Gehäuseaufnahmebereichs der Konzentratbehälteradapter an einem Konnektorgehäuse anbringbar ist. Der Gehäuseaufnahmebereich kann außen an dem die Öffnungen bildenden Material angeordnet sein.

Die Öffnungen können dabei die Verbindungsöffnungen und/oder die Befüllöffnung sein.

Die Befüllöffnung und die Verbindungsöffnungen können Durchgangsöffnungen sein und/oder können einen kreisförmigen Querschnitt aufweisen. Die Befüllöffnung kann einen größeren Durchmesser als die Verbindungsöffnungen aufweisen und zylinderförmig ausgebildet sein. Insbesondere kann die Befüllöffnung einen konstanten Innendurchmesser aufweisen. Die Verbindungsöffnungen können ebenfalls abschnittweise oder vollständig zylinderförmig sein, sie können sich in ihrem Radius entlang ihres Verlaufs auch ändern. Die Mittellinie der, vorzugsweise zylinderförmigen, Befüllöffnung kann dabei eine erste Gerade definieren. Die Mittelpunkte der, vorzugsweise im Querschnitt kreisförmigen, Verbindungsöffnungen können dabei zwei Punkte auf einer zweiten Geraden bilden und diese zweite Gerade definieren.

Die Mittellinie der, vorzugsweise zylinderförmigen, Verbindungsöffnungen können eine dritte und vierte Gerade definieren. Dabei können die dritte und vierte Gerade zueinander parallel und/oder parallel zu der ersten Geraden ausgebildet sein. Die erste und/oder dritte und/oder vierte Gerade können weiter im Wesentlichen senkrecht zu der zweiten Geraden ausgebildet sein. Die Verbindungsöffnungen können dabei sich auf der dritten, beziehungsweise vierten, Geraden gegenüberliegende Öffnungen aufweisen. Die Querschnittsflächen der sich gegenüberliegenden Öffnungen kann dabei voneinander verschieden sein, wobei die die Öffnung der Verbindungsöffnungen, welche in Richtung eines an dem Konzentratbehälteradapter angebrachten Konzentratbeutels weist, eine kleinere Querschnittsfläche aufweisen kann, als die gegenüberliegende Öffnung. Die sich gegenüberliegenden Öffnungen der Durchgangsöffnungen können dabei aber auch senkrecht zu der dritten, beziehungsweise vierten, Geraden beabstandet sein. Mit anderen Worten kann ein durch die Durchgangsöffnung strömendes Fluid zu zwei sich gegenüberliegenden Seiten ein-, beziehungsweise ausströmen, und wird zwischen den zwei Öffnungen innerhalb der jeweiligen Durchgangsöffnung umgelenkt.

Der Gehäuseaufnahmebereich ist außen an dem die Öffnungen bildenden Material angeordnet ist. Mit anderen Worten kann der Gehäuseaufnahmebereich von der Befüllöffnung und den Verbindungsöffnungen beabstandet sein. Beabstandet meint in diesem Fall, dass der Gehäuseaufnahmebereich nicht in der Befüllöffnung und/oder den Verbindungsöffnungen ausgebildet ist. Der Gehäuseaufnahmebereich kann dabei insbesondere nicht zwischen der Befüllöffnung und den Verbindungsöffnungen liegen. Insbesondere kann jeder Bereich des Gehäuseaufnahmebereichs weiter von dem Mittelpunkt der Befüllöffnung und/oder zumindest einer der Verbindungsöffnungen wegliegen, als die Länge der Verbindungslinie zwischen den Mittelpunkten der Befüllöffnung und zumindest einer der Verbindungsöffnungen. Zudem kann der jeder Punkt des Gehäuseaufnahmebereichs derart angeordnet sein, so dass der Abstand zwischen Mittelpunkt Befüllöffnung und zumindest dem Mittelpunkt einer der Verbindungsöffnungen kleiner ist als der Abstand zwischen Gehäuseaufnahmebereich und Mittelpunkt der Befüllöffnung zusammen mit dem Abstand zwischen Gehäuseaufnahmebereich und Mittelpunkt einer Verbindungsöffnung. Dabei kann der Gehäuseaufnahmebereich beispielsweise in einem Teilbereich um die Befüllöffnung herum ausgebildet sein. Die erste, dritte und vierte Gerade können weiter auf einer Ebene liegen. Zudem kann die erste Gerade die zweite Gerade im Wesentlichen senkrecht schneiden.

Nach einer Weiterbildung des Konzentratbehälteradapters kann der Aufnahmebereich der Öffnungskontur der Befüllöffnung folgen. Beispielsweise kann sich der Aufnahmebereich bogenförmig um die Befüllöffnung erstrecken und eine erste Kraftaufnahmefläche aufweisen, die in einem Winkel von 120 bis 60 Grad, vorzugsweise 110 bis 70 Grad, noch bevorzugter 90 Grad, zu der ersten Geraden ausgebildet ist, sowie eine Randfläche aufweisen, die derart ausgebildet ist, dass der Konzentratbehälteradapter relativ zu dem Konnektorgehäuse fixiert ist.

Nach einer Weiterbildung des Konzentratbehälteradapters kann der Gehäuseaufnahmebereich sich rahmenartig über zwei Längsstreben zu zwei, vorzugsweise parallel zu der ersten Geraden, gegenüberliegenden Seiten des Befestigungskörpers von dem Hauptkörper wegerstrecken und wenigstens eine erste Querstrebe aufweisen, welche die Längsstreben verbinden.

Diese Querstrebe kann zur Kraftaufnahme dienen, wenn der Konzentratbehälter an der Dialysevorrichtung angehängt wird. In einer Weiterbildung können zumindest zwei Querstreben die Längsstreben verbinden. Die zweite Querstrebe kann zum Halten oder Bewegen des Konzentratbehälters dienen, insbesondere kann die erste Querstrebe mittels manuellem oder automatischen Bewegen der zweiten Querstrebe in eine Halterung an der Dialysevorrichtung schiebbar sein. Die erste und/oder zweite Querstrebe können dabei bogenförmig ausgebildet sein. Insbesondere kann die zweite Querstrebe sich bogenförmig zwischen den Schnittpunkten der ersten Querstrebe mit den zwei Längsstreben erstrecken. Die zweite Querstrebe kann dabei klappbar oder faltbar ausgebildet sein, so dass die zweite Querstrebe während des Tragens von einem Pflegepersonal ausgeklappt ist und in einer Position, in welcher der Konzentratbehälteradapter auf dem Konnektorgehäuse angebracht ist, eingeklappt werden kann, so dass sich die zweite Querstrebe dem Konnektorgehäuse annähert.

Nach einer Weiterbildung des Konzentratbehälteradapters kann die erste Querstrebe einen Vorsprung und/oder eine Einkerbung aufweisen, die derart eingerichtet ist, dass sie einen Bewegungsfreiheitsgrad des Konzentratbehälteradapters beschränkt, wenn dieser an einem Konnektorgehäuse angebracht ist.

Insbesondere kann der Vorsprung und/oder die Einkerbung ein Verschieben des Konzentratbehälteradapters auf dem Konnektorgehäuse parallel zu Richtung der Querstrebe verhindern.

Nach einer Weiterbildung des Konzentratbehälteradapters kann dieser ein Konzentratbehälterrastelement am Befestigungskörper, das derart eingerichtet ist, dass es zumindest einen Bewegungsfreiheitsgrad des Konzentratbehälteradapters beschränkt, wenn dieses an einem Konnektorgehäuse angebracht ist, aufweisen.

Insbesondere kann das Konzentratbehälterrastelement ein Kippen des Konzentratbehälteradapters um die erste Querstrebe verhindern.

Erfindungsgemäß weist das Konnektorgehäuse zur Anbindung an eine Blutbehandlungsvorrichtung, zumindest einen Behälteraufnahmebereich, der derart ausgebildet ist, dass er einen Konzentratbehälteradapter, vorzugsweise nach einem der vorangegangenen Aspekte, an einem unteren Bereich des Konnektorgehäuses aufnehmen kann, und Konnektorverbindungselemente an einer Unterseite des Konnektorgehäuses auf, welche aus dem Konnektorgehäuse verfahrbar sind, und derart eingerichtet sind, dass eine fluidische Verbindung mit dem Konzentratbehälteradapter herstellbar ist.

Der untere Bereich des Konnektorgehäuses meint dabei einen Bereich des Konnektorgehäuses, welcher von einer Draufsicht senkrecht zu einer Oberseite des Konnektorgehäuses nicht zu sehen ist. Mit anderen Worten meint der untere Bereich des Konnektorgehäuses sowohl eine Unterseite, welche sich parallel zu der Oberseite des Konnektorgehäuses erstreckt sowie eine Stirnfläche, welche sich von der Unterseite des Konnektorgehäuses weiter von der Oberseite des Konnektorgehäuses wegerstreckt. Die Konnektion des Konzentratebeutels, welcher in dem Konzentratbehälteradapter aufgenommen ist, erfolgt dabei von oben, das heißt in einer Richtung, welche sich von der Oberseite des Konnektorgehäuses wegerstreckt.

Nach einer Weiterbildung des Konnektorgehäuses kann der Behälteraufnahmebereich eine rillenförmige Aussparung auf der Oberseite des Konnektorgehäuses sein, welche sich auf einer ersten Geraden erstreckt.

Dabei kann die rillenförmige Aussparung im Querschnitt halbkreisförmig ausgebildet sein. Ebenso kann die rillenförmige Aussparung im Querschnitt rechteckig, mit Einführschrägen zum leichten Einsätzen des Konzentratbehälteradapters, ausgebildet sein.

Nach einer Weiterbildung des Konnektorgehäuses kann der rillenförmigen Aussparung ein Beschränkungselement zugeordnet sein, welches sich auf einer zweiten, von der ersten verschiedenen Richtung erstreckt.

Das Beschränkungselement kann dabei in der rillenförmigen Aussparung ausgebildet sein. Das Beschränkungselement kann beispielsweise eine rechteckige oder halbkreiskugelförmige Erhebung in der rillenförmigen Aussparung sein. Dabei erstreckt sich das Beschränkungselement in einer Richtung senkrecht zu der Oberseite des Konnektorgehäuses, so dass eine Kraft, welche parallel zu der Oberseite des Konnektorgehäuses wirkt, aufgenommen werden kann. Mit anderen Worten kann der Konzentratbehälteradapter bei einem Einsetzen in die rillenförmige Aussparung nicht mehr verschoben werden.

Nach einer Weiterbildung des Konnektorgehäuses kann ein Konnektorgehäuserastelement an einer Unterseite des Konnektorgehäuses ausgebildet sein.

Das Konnektorgehäuserastelement kann insbesondere an einer Stirnfläche, welche sich von der Unterseite des Konnektorgehäuses nach unten erstreckt, angeordnet sein, so dass bei einem Einlegen des Konzentratbehälteradapters dieser durch das Rastelement an einem Verkippen in der rillenförmigen Aussparung gehindert werden kann.

Nach einer Weiterbildung kann das Konnektorgehäuses weiter ein Türelement, mit einer ersten Haltevorrichtung an einer Seite des Türelements, eine zweite Haltevorrichtung zur beweglichen Befestigung des Türelements an dem Konnektorgehäuse, aufweisen wobei der Behälteraufnahmebereich zur Aufnahme des Konzentratbehälteradapters auf dem Türelement ausgebildet ist, wobei das Türelement derart um die zweite Haltevorrichtung verschwenkbar ist, so dass in einer ersten Position die der ersten Seite des Türelements gegenüberliegenden zweiten Seite unter das Konnektorgehäuse bewegbar ist, und in einer zweiten Position sich das Türelement von dem Konnektorgehäuse wegerstreckt.

Nach einer Weiterbildung kann ein Wandmaterial des Behälteraufnahmebereichs flach ausgebildet sein. Alternativ kann der Behälteraufnahmebereich auch eine Kontur aufweisen. Diese Oberflächenkontur kann als Auflage für den Konzentratbehälteradapter dienen. Der Gehäuseaufnahmebereich des Konzentratbehälteradapters kann eine entsprechende Kontur aufweisen. Beispielsweise kann die Kontur wellenförmig sein oder zwei Flächen aufweisen, die sich unter einem Winkel kleiner 180° treffen. Eine solche Kontur kann dazu dienen, die Positionierung des Adapters, wenn er eingehängt wird, genauer zu definieren.

Das Türelement kann dabei bogenförmig ausgebildet sein, und ein Mittelpunkt der Bogenform kann in der ersten Position des Türelements unter dem Konnektorgehäuse liegen.

Nach einer Weiterbildung des Konnektorgehäuses kann ein erstes Eingriffelement, das an der zweiten Seite des Türelements ausgebildet ist, ausgebildet sein. Ebenso kann ein zweites Eingriffselement, das an dem Konnektorgehäuse ausgebildet ist, ausgebildet sein. Somit kann in der ersten Position des Türelements das erste Eingriffelement mit dem zweiten Eingriffelement eingreifen. Hierdurch kann ein haptisches Feedback an das Pflegepersonal entstehen. Zudem kann ein leichtes Zurückschwenken der Türe verhindert werden. Ebenso können weitere oder alternative Eingriffselemente vorgesehen sein, welche ineinandergreifen, wenn das Türelement die zweite Position erreicht.

Nach einer Weiterbildung kann das Konnektorgehäuse eine Erfassungseinheit zum Erfassen eines Konzentratbehälteradapters aufweisen.

Dabei kann ein Teil der Erfassungseinheit an dem Konnektorgehäuse ausgebildet sein und ein zweiter Teil der Erfassungseinheit an dem Konzentratbehälteradapter oder dem Konzentratbeutel, welcher mit dem ersten Teil der Erfassungseinheit wechselwirkt. Dabei kann die Erfassungseinheit mit einer Steuereinheit verbunden sein, so dass eine Konnektion des Konzentratbehälteradapters, beziehungsweise Konzentratbeutels erst erfolgt, wenn durch die Erfassungseinheit ein Vorhandensein eines Konzentratbehälteradapters, beziehungsweise eines Konzentratbeutels positiv erfasst wurde. Mit anderen Worten kann sowohl ein Vorhandensein, die richtige Position des Konzentratbeutels als auch ein Vorhandensein des richtigen Konzentratbeutels erfasst werden.

Nach einer Weiterbildung des Konnektorgehäuses kann die Erfassungseinheit eine Lichtsendeeinheit sowie eine Lichtempfangseinheit zum Erfassen eines Vorhandenseins an einer bestimmten Position einer Reflexionsgeometrie sein.

Dabei kann mit Hilfe des Snelliusschen Brechungsgesetzes genau bestimmt werden, wann die Lichtempfangseinheit ein Reflexion detektieren soll. Wird der Konzentratbehälteradapter in einer bestimmten Position eingelegt und weist dieser einen Bereich, das heißt eine Reflexionsgeometrie, auf, auf welchen die Lichtsendeeinheit gerichtet ist, so kann die Richtungsänderung auf Basis des Materials des Bereichs, auf welchen die Lichtsendeeinheit gerichtet ist, berechnet werden. Mit anderen Worten kann die Reflexionsgeometrie nur dann einen Lichtstrahl zu der Lichtempfangseinheit zurücksenden, wenn sich der Konzentratbehälteradapter mit der Reflexionsgeometrie in der richtigen Position relativ zu dem Konnektorgehäuse befindet.

Nach einer Weiterbildung des Konnektorgehäuses kann die Erfassungseinheit ein Farbsensor sowie eine Lichtquelle sein, welche an der Unterseite des Konnektorgehäuses angeordnet sind.

Bei dieser Weiterbildung kann der Konzentratbehälteradapter eine Farbkodierung aufweisen. Dabei können die Farbsensoren auf Interferenzbasis arbeiten und beispielsweise Photodioden mit integrierten Farbfiltern sein. Auch hier ist zur Auswertung eine Steuereinheit ausgebildet, so dass erst bei einem positiven Detektieren eine Konnektion des Konzentratbehälteradapters erfolgt.

Nach einer Weiterbildung des Konnektorgehäuses kann die Erfassungseinheit ein Radar-Sensor zur Bewegungs- und Lageerkennung des Konzentratbehälteradapters sein.

Nach einer Weiterbildung des Konnektorgehäuses kann die Erfassungseinheit zumindest ein Mikrotaster sein.

Hierbei weist das Konnektorgehäuse an einer Position, an welcher sich der Konzentratbeutel, beziehungsweise der Konzentratbehälteradapter in einer korrekten Position befindet, zumindest einen, vorzugsweise aber mehrere Mikrotaster auf.

Nach einer Weiterbildung weist eine Aufnahmeeinrichtung zur Aufnahme eines Konzentratbehälteradapters, vorzugsweise nach einem der vorangegangenen Aspekte, einen Aufnahmebereich, mit Befestigungselementen zur Befestigung des Konzentratbehälteradapters, einen Montagebereich, mit Montageelementen zur Montage des Aufnahmebereichs an einem Konnektorgehäuse, vorzugsweise nach einem der vorangegangenen Aspekte, einen Verfahrbereich, mittels welchem der Abstand zwischen dem Aufnahmebereich und dem Montagebereich derart veränderbar ist, dass der Aufnahmebereich von einem dem Konnektorgehäuse entfernten Bereich zu einem dem Konnektorgehäuse nahen Bereich verfahrbar ist, auf.

Nach einer Weiterbildung der Aufnahmeeinrichtung kann der Verfahrbereich als ein Teleskopmechanismus oder als Klappmechanismus ausgebildet sein und/oder der Verfahrbereich auf einer Geraden verfahren werden, die einen Winkel von 20 bis 50 Grad, vorzugsweise 30 bis 40 Grad, zwischen einer zu einer Bodenfläche in einem Zustand, in dem die Aufnahmeeinrichtung mit dem Konnektorgehäuse verbundenen ist, parallelen Fläche aufspannt.

Nach einer Weiterbildung weist eine Aufnahmeeinrichtung zur Aufnahme eines Konzentratbehälteradapters, vorzugsweise nach einem der vorangegangenen Aspekte, einen Montagebereich, mit Montageelementen zur Montage des Aufnahmebereichs an einem Konnektorgehäuse, vorzugsweise nach einem der vorangegangenen Aspekte, einen Aufnahmebereich auf, in welchen der Konzentratbehälteradapter einlegbar ist, sowie einen Gleitbereich auf, der derart eingerichtet und zu einer Bodenfläche, in einem Zustand, in dem der Aufnahmebereich an dem Konnektorgehäuse montiert ist, geneigt ist, dass ein Konzentratbehälter auf einer Linie zu dem Konnektorgehäuse verschiebbar ist, wobei der Aufnahmebereich derart schwenkbar zu dem Gleitbereich ausgebildet ist, so dass bei einem Verschwenken des Aufnahmebereichs, ein in den Aufnahmebereich eingelegter Konzentratbehälter aus dem Aufnahmebereich über den Gleitbereich dem Konnektorgehäuse verschiebbar sein kann.

Nach einer Weiterbildung des Aufnahmebereichs kann der Gleitbereich aus zwei parallel verlaufenden Schienenelemente ausgebildet sein und der Aufnahmebereich bogenförmig zwischen Endabschnitten der Schienenelemente ausgebildet sein.

Erfindungsgemäß kann ein Konnektionssystem ein Konnektorgehäuse nach einem der vorangegangen Aspekte, sowie einen Konzentratbehälteradapter nach einem der vorangegangen Aspekte aufweisen.

Nach einer Weiterbildung des Konnektionssystems kann diese eine Aufnahmeeinrichtung nach einem der Ansprüche 18 bis 20 aufweisen.

Erfindungsgemäß kann ein Konzentratbehältersystem einen Konzentratbehälteradapter nach einem der vorangegangenen Aspekte, einen Konzentratbehälter, der mit einem Trockenkonzentrat befüllbar ist, und/oder eine Konzentratzuführleitung, welche eine Konzentratlösung aus dem Konzentratbehälter einer Blutbehandlungsvorrichtung, insbesondere einer Dialysemaschine zuführen kann, aufweisen.

Durch den Konzentratbehälteradapter und das Konnektorgehäuse ist es möglich den Konzentratbehälteradapter leicht, das heißt mit wenigen Handhabungsschritten mit einer Blutbehandlungsvorrichtung, insbesondere einer Dialysemaschine, zu verbinden. Insbesondere kann der Konzentratbehälteradapter an der Blutbehandlungsvorrichtung angebracht werden, während noch ein Reinigungsschritt der Konnektionstüllen erfolgt. Durch die Ausbildung des Konzentratbehälteradapters ist es zudem möglich, ein Verrutschen des Konzentratbehälteradapters relativ zu der Blutbehandlungsvorrichtung, insbesondere der Dialysemaschine, zu verhindern. Durch den erfindungsgemäßen Konzentratbehätleradapter und Konnektorgehäuse kann die Sicherheit des Patienten gewährleistet werden.

Die vorstehend beschriebenen Merkmale und Funktionen der vorliegenden Erfindung sowie weitere Aspekte und Merkmale werden nachfolgend anhand einer detaillierten Beschreibung von bevorzugten Ausführungsformen unter Bezugnahme auf die beigefügten Figuren weiter beschrieben. In den Figuren sind gleiche Merkmale/Elemente und Merkmale/Elemente mit der gleichen Funktion mit den gleichen Bezugszeichen gekennzeichnet.

### Kurzbeschreibung der Zeichnungen

Hierbei zeigt:
- Fig. 1: einen Konzentratbehälteradapter nach einer ersten Ausführungsform;
- Fig. 2: einen Konzentratbehälteradapter nach einer zweiten Ausführungsform;
- Fig. 3: einen Konzentratbehälteradapter nach einer dritten Ausführungsform;
- Fig. 4: ein Konnektorgehäuse nach einer ersten Ausführungsform;
- Fig. 5: ein Konnektorgehäuse nach einer zweiten Ausführungsform;
- Fig. 6: ein Konnektorgehäuse nach einer dritten Ausführungsform;
- Fig. 7: eine Prinzipdarstellung einer Erfassungseinheit nach einer ersten Ausführungsform;
- Fig. 8: eine Prinzipdarstellung einer Erfassungseinheit nach einer zweiten Ausführungsform;
- Fig. 9: eine Prinzipdarstellung einer Erfassungseinheit nach einer dritten Ausführungsform;
- Fig. 10: eine Prinzipdarstellung einer Erfassungseinheit nach einer vierten Ausführungsform;
- Fig. 11: eine Prinzipdarstellung einer Aufnahmeeinrichtung nach einer ersten Ausführungsform;
- Fig. 12: eine Prinzipdarstellung einer Aufnahmeeinrichtung nach einer zweiten Ausführungsform.

### Detaillierte Beschreibung eines Ausführungsbeispiels

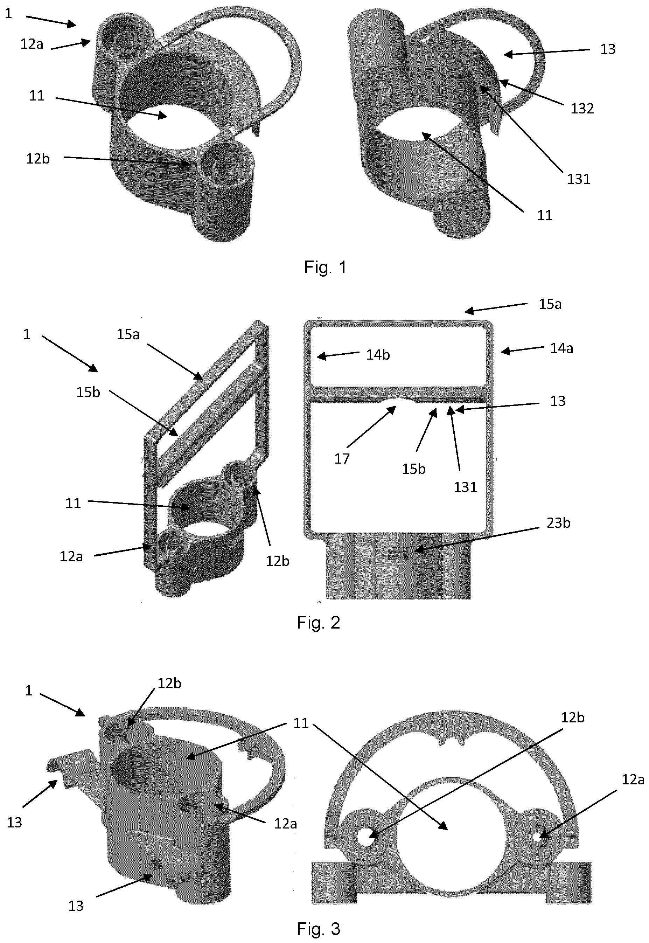

Bezugnehmend auf Fig. 1 wird nachstehend eine erste Ausführungsform erläutert werden. Dabei zeigt Fig. 1 einen Konzentratbehälteradapter nach einer ersten Ausführungsform. Der Konzentratbehälteradapter weist einen Befestigungskörper 1 auf. An dem Befestigungskörper 1 kann ein Konzentratbehälter, insbesondere ein Konzentratbeutel, welcher mit Trockenkonzentrat befüllbar ist, angebracht werden. Der Konzentratbeutel kann aus einem flexiblem Material wie beispielsweise PVC, PVP oder Polyethylen ausgebildet sein. Der Konzentratbeutel, vorteilhafterweise noch ohne Trockenkonzentrat, kann mit dem Konzentratbehälteradapter verschweißt werden.

Zur Vorbereitung einer Dialysebehandlung wird der Konzentratbehälteradapter mit an diesem angebrachten Konzentratbehälter, welcher nun mit Trockenkonzentrat befüllt ist, an einer Dialysemaschine befestigt. Das Trockenkonzentrat ist bei einer Bicarbonatdialyse Bicarbonatkonzentrat. Der Befestigungskörper 1 weist eine Befüllöffnung 11 auf. Über diese Befüllöffnung 11 kann das Trockenkonzentrat in den Konzentratbehälter eingefüllt werden. Die Befüllöffnung 11 spannt dabei eine Ebene auf. Senkrecht zu dieser Ebene erstreckt sich die erste Gerade. Die Befüllöffnung 11 kann wie in der in Fig. 1 gezeigten Ausführungsform eine sich erstreckende rohrförmiger, oder ringförmiger Abschnitt sein.

Weiter weist der Befestigungskörper 1 eine erste Verbindungsöffnung 12a sowie eine zweite Verbindungsöffnung 12b auf. Diese Verbindungsöffnungen 12a und 12b spannen eine zweite Gerade auf. An den Verbindungsöffnungen 12a und 12b können jeweils ein Schlauch angebracht sein, welcher in den Konzentratbeutel ragt. An zumindest einem Schlauch, insbesondere dem Ansaugende, kann zudem ein Filter angebracht sein. Die Befüllöffnung 11 wird nach dem Einfüllen mit einem Trockenkonzentrat vorzugsweise mit einer Schutzfolie verschlossen, beispielsweise verschweißt oder verklebt. Ebenso sind die erste Verbindungsöffnung 12a und die zweite Verbindungsöffnung 12b vor einer Behandlung mit einer Schutzvorrichtung, beispielsweise einer Schutzfolie, verschlossen. Vor Beginn der Behandlung, genauer, bevor Reinwasser aus der Dialysemaschine in den Konzentratbeutel über den Konzentratbehälteradapter eingefüllt werden kann, wird die Schutzvorrichtung von den Verbindungsöffnungen 12a, 12b entfernt.

Während der Behandlung wird über die erste Verbindungsöffnung 12a Reinwasser aus der Dialysemaschine in den Konzentratbeutel eingeleitet. In dem Konzentratbeutel findet eine Vermischung des Trockenkonzentrats mit dem Reinwasser statt. Anschließend wird die Konzentratlösung, nun in flüssiger Form, über die zweite Verbindungsöffnung 12b ausgeleitet. Hierfür kann in dem Konzentratbeutel, verbunden mit der zweiten Verbindungsöffnung 12b, ein Schlauch angeordnet sein, welcher sich von der zweiten Verbindungsöffnung 12b bis nahe an dem der zweiten? Verbindungsöffnung 12b gegenüberliegenden Endbereich des Konzentratbeutels erstreckt. Der Konzentratbehälteradapter, an welchem ein Konzentratbeutel angebracht ist, wird vor einer Behandlung an der Dialysemaschine angebracht.

Zur Handhabung kann der Konzentratbehälteradapter, wie in Fig. 1 dargestellt, einen Tragegriff haben, welcher in dieser Ausführungsform vorzugsweise einklappbar ausgebildet ist. Hierfür weist der Konzentratbehälteradapter einen Gehäuseaufnahmebereich 13 am Befestigungskörper 1 auf. Über diesen Gehäuseaufnahmebereich 13 wird der Konzentratbehälteradapter derart an der Dialysemaschine angebracht, dass der Konzentratbehälteradapter relativ zu der Dialysemaschine nicht mehr bewegbar ist. Mit anderen Worten werden durch diesen Gehäuseaufnahmebereich 13 die Kraftübertragung in vorzugsweise allen Freiheitsgraden ermöglicht. Über diesen Gehäuseaufnahmebereich 13 wird insbesondere auch die Gewichtskraft des Konzentratbehälteradapters mit Konzentratbeutel auf die Dialysemaschine, genauer auf ein für die Aufnahme des Konzentratbehälteradapters vorgesehenes Konnektorgehäuse 10, übertragen.

Der Gehäuseaufnahmebereich 13 ist dabei um die Befüllöffnung 11 ausgebildet. Der Gehäuseaufnahmebereich 13 kann dabei, wie in der in Fig. 1 gezeigten Ausführungsform, bogenförmig um die Befüllöffnung 11 ausgebildet sein. Dabei erstreckt sich eine erste Kraftaufnahmefläche 131 von der ersten Befüllöffnung 11 weg. Über diese erste Kraftaufnahmefläche 131 kann insbesondere die Gewichtskraft des Konzentratbeutels auf das Konnektorgehäuse 10 übertragen werden. Die erste Kraftaufnahmefläche 131 bildet dabei in der hier dargestellten Ausführungsform einen Winkel von im Wesentlichen 90 Grad zu der ersten Geraden aus. Zur Verhinderung, dass sich der Konzentratbehälteradapter relativ zu dem Konnektorgehäuse 10 verschieben kann, weist der Aufnahmeberiech eine Randfläche 132 auf.

Die Randfläche 132 erstreckt sich von der ersten Kraftaufnahmefläche 131 rechtwinklig in Richtung in Bezug zu einer an dem Konnektorgehäuse 10 angebrachten Lage, nach unten, das heißt entgegen der Schwerkraftsrichtung. Da der Aufnahmebereich bogenförmig ausgebildet ist, kann dieser ebenso Kräfte aufnehmen, die senkrecht zu der Bogenform wirken. Fig. 2 zeigt eine weitere Ausführungsform des Konzentratbehälteradapters. Der Gehäuseaufnahmebereich 13 erstreckt sich in dieser Ausführungsform rahmenartig um die Befüllöffnung 11. Dabei erstrecken sich insbesondere zwei Längsstreben 14a, 14b zu zwei Seiten des Befestigungskörpers 1 weg. Dabei erstrecken sich die Längsstreben 14a, 14b insbesondere von zwei gegenüberliegende Seiten des Befestigungskörpers 1, genauer zu zwei Stellen, welche auf der zweiten Gerade liegen. Dabei schneidet die zweite Gerade einen Mittelpunkt der Befüllöffnung 11.

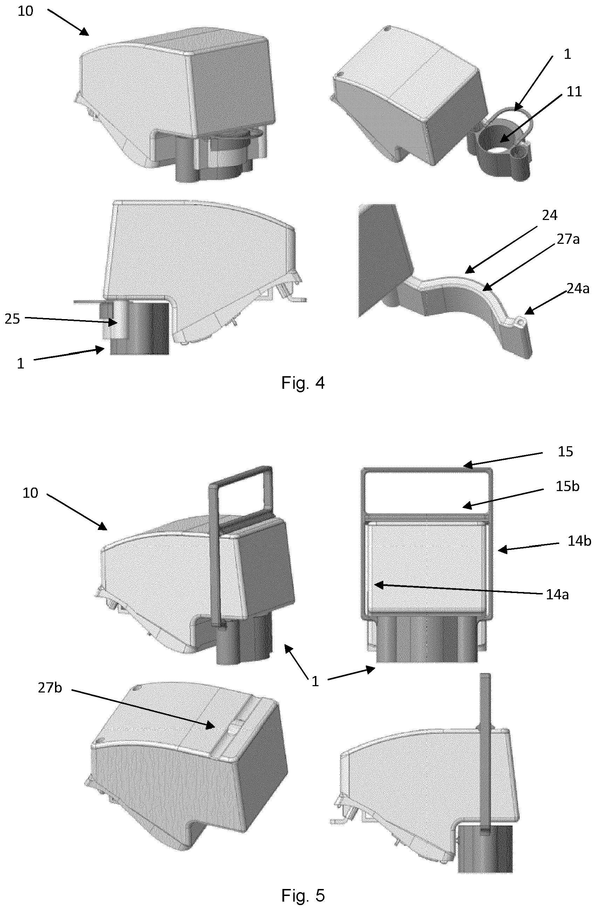

Damit kann vermieden werden, dass sich große Kippmomente bilden. Insbesondere wirkt bei einer Konnektion des Konzentratbeutels, das heißt einem Verbinden der dialyseseitigen Anschlüsse mit der ersten und zweiten Verbindungsöffnung 12a, 12b eine Kraft auf den Konzentratbehälteradapter. An einem von dem Hauptkörper entfernten Endbereich der Längsstreben 14a, 14b befinden sich zwei Querstreben 15a, 15b. Diese Querstreben 15a, 15b sind dabei parallel zu der zweiten Geraden ausgebildet. Die Querstreben 15a, 15b sind zueinander derart beabstandet ausgebildet, dass der Konzentratbehälteradapter, die erste, obere Querstrebe 15a umfassend, leicht getragen werden kann, beispielsweise in einem Abstand von 1,5 bis 10cm, vorzugsweise 2 bis 8 cm, ausgebildet sein. Über die untere, das heißt in Richtung der Befüllöffnung 11 liegenden, Querstrebe 15b findet die Kraftübertragung der Gewichtskraft auf ein Konnektorgehäuse 10 statt. Dabei weist das Konnektorgehäuse 10, wie in Fig. 5 gezeigt, eine rillenförmige Aufnahme als Behälteraufnahmebereich 27b auf. Das Konnektorgehäuse 10 kann dabei nach vorne konisch zulaufen. Mit anderen Worten kann der Umfang des Konnektorgehäuse an einer Vorderseite, über welche der Konzentratbehälteradapter zu Beginn aufgeschoben werden muss, geringer sein als an einer Stelle, an welcher der Konzentratbehälteradapter schließlich in die Rille eingesetzt werden kann.

Die untere Querstrebe 15b weist, wie in Fig. 2b dargestellt, eine Einkerbung 17 auf. Über die Einkerbung 17 kann der Konzentratbehälteradapter an der richtigen Stelle an dem Konnektorgehäuse 10 eingehängt werden. Zudem verhindert die Einkerbung 17 in der unteren Querstrebe 15b ein Verrutschen des Konzentratbehälteradapters relativ zu dem Konnektorgehäuse 10. Der Konzentratbehälteradapter weist weiter ein Konzentratbehälterrastelement 23a auf. Hierfür ist an einer Seitenfläche des Befestigungskörpers 1 eine im Querschnitt halbkreisförmige, sich erstreckende Kontur ausgebildet. Das Konnektorgehäuse 10, wie in Fig. 5 dargestellt, weist eine entsprechende Gegenkontur auf, welche mit dem Konzentratbehälterrastelement 23a eingreifen, genauer einrasten kann. Hierdurch können Kippmomente des Konzentratbehälteradapters aufgenommen werden.

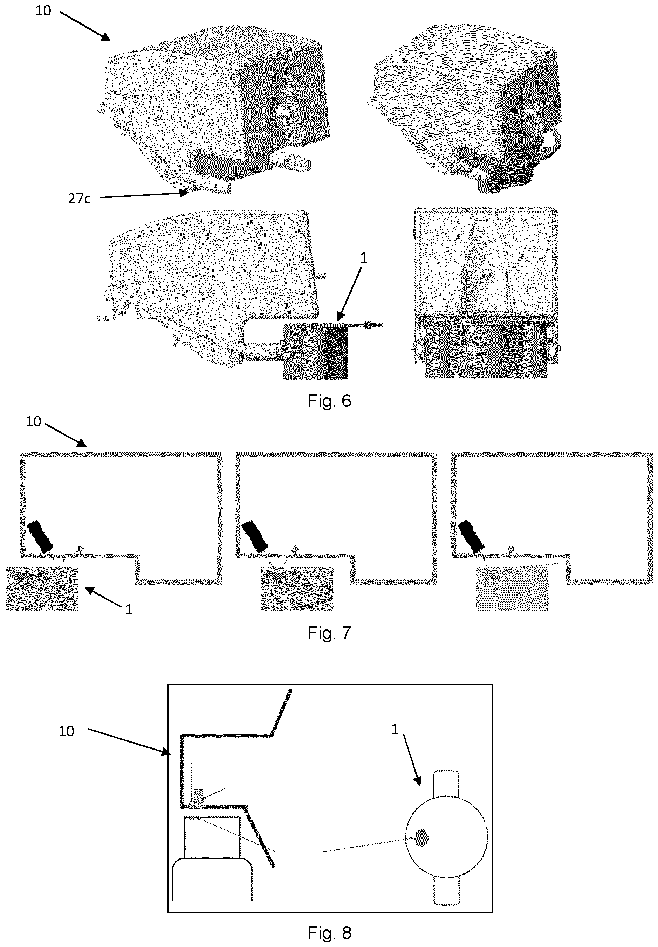

Fig. 3 zeigt eine weitere Ausführungsform eines Konzentratbehälteradapters. Hierbei ist der Gehäuseaufnahmebereich 13 zu zwei Seiten der Befüllöffnung 11 ausgebildet. Genauer sind zwei im Querschnitt halbkreisförmige Aufnahmebereiche ausgebildet, welche auf eine entsprechende Gegenkontur, etwa zylinderförmige und/oder konusförmige Zapfen aufschieben können. Die Aufnahmebereiche stehen damit auf einer Geraden, parallel zur zweiten Geraden, von dem Konzentratbehälteradapter seitlich hervor. Eine entsprechende Ausführungsform des Konnektorgehäuses 10 ist in Fig. 6 dargestellt. Die zapfenartigen Fortsätze dienen bei dieser Ausführungsform als Behälteraufnahmebereich 27c. Dabei kann das Konnektorgehäuse 10 an seiner Vorderseite einen zusätzlichen, dritten zapfenartigen Fortsatz aufweisen. Auf diesen Fortsatz kann der Konzentratbehälteradapter eingehängt werden. Hierfür kann der Konzentratbehälteradapter an seinem Tragegriff eine u-förmige Ausnehmung haben.

Fig. 4 zeigt perspektivische Ansichten eines Konnektorgehäuses 10 nach einer ersten Ausführungsform. Das Konnektorgehäuse 10 weist zur Aufnahme eines Konzentratbeutels, genauer eines Konzentratbehälteradapters, einen Behälteraufnahmebereich 27a auf. Um Reinwasser dem Konzentratbeutel zuführen zu können sowie die Konzentratlösung aus dem Konzentratbeutel der Dialysemaschine zuführen zu können, weist das Konnektorgehäuse 10 Anschlusstüllen auf, welche vorzugsweise verfahrbar, in dem Konnektorgehäuse 10 angeordnet sind, und damit von oben in den angebrachten Konzentratbehälteradapter einfahren können. Durch diese Anschlusstüllen kann damit eine fluidische Verbindung zwischen Konzentratbeutel und der Dialysemaschine hergestellt werden.

Bei der in Fig. 4 gezeigten Ausführungsform weist das Konnektorgehäuse 10 ein Türelement 24 auf. Das Türelement 24 kann relativ zu dem Konnektorgehäuse 10 verschwenkt werden. Hierfür weist das Türelement 24 einen Zylinder mit Bohrung auf. Das Konnektorgehäuse 10 weist beispielsweise einen dem Innendurchmesser der Bohrung entsprechenden zweiten Zylinder auf. Auf diesen kann die Türe gesteckt und anschließend verschraubt werden. Alternativ kann ein Scharniergelenk das Türelement 24 mit dem Konnektorgehäuse 10 verbinden. Der Behälteraufnahmebereich 27a ist an dem Türelement 24 ausgebildet, wobei ein Konzentratbehälteradapter der ersten Ausführungsform über die Randfläche 132 des Türelements 24 eingehängt werden kann. Der Behälteraufnahmebereich 27a, beziehungsweise das Türelement kann zur Erleichterung der Platzierung des Konzentratbehälteradapter Einfuhrschrägen aufweisen. Zusätzlich oder alternativ kann der Konzentratbehälteradapter Einfuhrschrägen aufweisen. Zum Einhängen des Konzentratbehälteradapters wird das Türelement 24 in eine offene Stellung gebracht.

Um den Konzentratbeutel aufnehmen zu können, ist das Türelement 24 bogenförmig ausgebildet. In geschlossener Stellung sind dabei beide Endbereiche des Türelement 24 unter dem Konnektorgehäuse 10 angeordnet. In der geschlossenen Stellung kann eine Kontaktfläche beider Endbereiche des Türelements 24 mit dem Konnektorgehäuse 10 bestehen. Hierfür kann der Endbereich des Türelements 24, welcher sich in der geöffneten Stellung nicht unter dem Konnektorgehäuse 10 befindet, ein erstes Eingriffelement 24a aufweisen. Das erste Eingriffelement 24a kann, wie in der in Fig. 4 gezeigten Ausführungsform eine Einkerbung 17 auf der Oberseite des Türelements 24 sein. Das Konnektorgehäuse 10 weist eine entsprechende Gegenkontur, beispielsweise in Form eines durch eine Feder beweglich gelagerten Kugelelements. Befindet sich das Türelement 24 unter dem Konnektorgehäuse 10, wird das Kugelelement in die Einkerbung 17 gedrückt.

Zum Erfassen, ob sich ein Konzentratbeutel, beziehungsweise ein Konzentratbehälteradapter an dem Konnektorgehäuse 10 befindet, kann das Konnektorgehäuse 10 eine Erfassungseinheit aufweisen. Hierfür können, wie in einer Prinzipdarstellung in Fig. 7 dargestellt ist, eine Lichtquelle als Sender sowie ein Lichtempfänger an dem Konnektorgehäuse 10 ausgebildet sein. Sender und Empfänger können dabei an einer Unterseite des Konnektorgehäuses 10, unter welcher der Konzentratbehälteradapter aufgenommen werden kann, ausgebildet sein. Der Konzentratbehälteradapter weist an einer Oberfläche eine Reflexionsgeometrie auf. Sendet die Lichtquelle einen Lichtstrahl in einer bestimmten Richtung aus, so wird der Lichtimpuls in entsprechender Stärke nur an dem Lichtempfänger aufgenommen, wenn der Konzentratbehälteradapter an einer vorgegebenen Position relativ zu dem Konnektorgehäuse 10 angeordnet ist.

Die Reflexionsgeometrie auf dem Konzentratbeuteladapter kann dabei variieren, beispielsweise in Abhängigkeit von dem Trockenkonzentrat, welches sich in dem Konzentratbeutel befindet. Damit wird nicht nur sichergestellt, dass sich der Konzentratbeuteladapter an der richtigen Position befindet, vielmehr wird auch sichergestellt, dass das für eine bestimmte Behandlung vorgesehene Trockenkonzentrat bereitgestellt wurde. Wird festgestellt, dass sich ein falscher Konzentratbehälteradapter oder sich dieser an einer falschen Position erfüllt, mit andere Worten, die Reflexionsgeometrie nicht durch das Empfangen an der Empfangseinheit detektiert, kann ein Behandlungsbeginn verhindert werden. Beispielsweise kann dem Pflegepersonal eine Nachricht, beispielsweise in Form eines Alarms, präsentiert werden.

Das Konnektorgehäuse 10 kann in einer weiteren Ausführungsform als Erfassungseinheit einen Farbsensor als Empfänger sowie ein Lichtquelle als Sendeinheit aufweisen. Entsprechend kann wie in der in Fig. 8 gezeigten Prinzipdarstellung der Konzentratbeutel oder der Konzentratbehälteradapter eine Farbmarkierung aufweisen. Dabei kann je nach farblicher Kodierung erfasst werden, welche Art von Trockenkonzentrat, welcher Konzentratbeuteltyp, beispielsweise unterschieden nach der Füllmenge, sowie eine korrekte Positionierung erfasst werden. Auch bei dieser Ausführungsform können Lichtquelle sowie Farbsensor an einer Unterseite des Konnektorgehäuses 10 ausgebildet sein. Als weitere Ausführungsform kann ein Radarsensor an dem Konnektorgehäuse 10 angeordnet sein, und die Lage sowie das Vorhandensein des Konzentratbeuteladapters, beziehungsweise Konzentratbeutels erfassen.

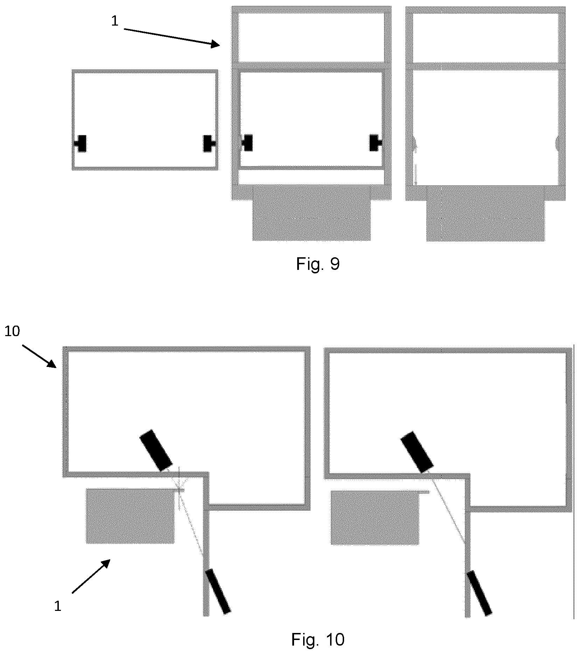

Fig. 9 zeigt eine Prinzipdarstellung einer weiteren Ausführungsform der Erfassungseinheit. Auch bei dieser Ausführungsform weist das Konnektorgehäuse 10 eine Sendeeinheit sowie Empfangseinheit auf. Die Sendeeinheit ist hier eine Lichtquelle. Die Empfangseinheit ist derart an dem Konnektorgehäuse 10 angeordnet, dass sie nur einen Lichtimpuls empfängt, wenn sich der richtige Konzentratbeutel, beziehungsweise Konzentratbehälteradapter an der richtigen Stelle befindet. Dies wird aufgrund der charakteristischen Brechzahl des verwendeten Materials sichergestellt. Dabei wird die Brechzahl des Konzentratbeutels oder Konzentratbehälteradapters als Grundlage für den erwarteten Reflexionswinkel genommen. Wird an einer entsprechend positionierten Empfangseinheit kein zuvor definierter Lichtimpuls empfangen, sind die zuvor genannten Bedingungen des richtigen Konzentratbeutels und/oder Position desselben nicht erfüllt. Die Empfangseinheit kann dabei, wie in Fig. 9 dargestellt, an einer der Unterseite des Konnektorgehäuses 10 in einem Winkel gegenüberliegenden Seite des Konnektorgehäuses 10 ausgebildet sein.

Fig. 10 zeigt eine weitere Ausführungsform der Erfassungseinheit. Hierbei weist das Konnektorgehäuse 10 an einer Position, an welcher sich der Konzentratbeutel, beziehungsweise der Konzentratbehälteradapter in einer korrekten Position befindet, zumindest einen, vorzugsweise aber mehrere Mikrotaster auf. Wie in Fig. 10 gezeigt, kann der Konzentratbehälteradapter zwei, an den Innenseiten der Längsstreben 14a, 14b angeordnete Vorsprünge aufweisen. Der Mikrotaster an den Seitenflächen des Konnektorgehäuses 10 kann direkt hinter einer Elastomerschicht angeordnet werden, so dass bei einem Eindrücken dieser durch die Vorsprünge der Mikrotaster ausgelöst wird. Die Kodierung verschiedener Konzentratbeutel kann dabei über die Position der Mikrotaster an dem Gehäuse erfolgen.

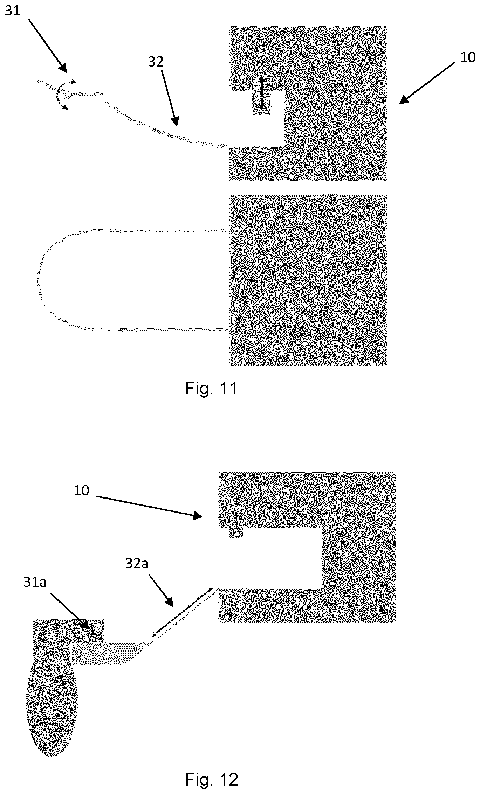

Fig. 11 zeigt eine Prinzipdarstellung einer Aufnahmeeinrichtung 3 nach einer ersten Ausführungsform. In der Aufnahmeeinrichtung 3 kann dabei ein Konzentratbehälteradapter aufgenommen werden und die Aufnahmeeinrichtung selbst kann über einen Montagebereich an dem Konnektorgehäuse 10 angebracht werden. Zudem weist die Aufnahmeeinrichtung 3 einen Gleitbereich 32 sowie einen Aufnahmebereich 31 auf. Damit kann der Aufnahmebereich 31, in welchem ein Konzentratbehälteradapter aufgenommen wird, über den Gleitbereich 32, dem Konnektorgehäuse 10 zugeführt werden. Das Pflegepersonal kann somit den Konzentratbehälteradapter mit Konzentratbeutel in einem Bereich einsetzen, welcher leicht zu erreichen ist. Die Zuführung des Konzentratbehälteradapter selbst, erfolgt dann über den Gleitbereich 32.

Wie in Fig. 11 dargestellt ist, ist der Gleitbereich 32 hier als Stangenrutsche, beispielweise als starres Element, ausgebildet. Der Gleitbereich 32 kann aus zwei parallel verlaufenden Schienenelementen ausgebildet sein und der Aufnahmebereich 31 bogenförmig zwischen Endabschnitten der Schienenelemente ausgebildet sein. Über das starre Element kann der Konzentratbehälteradapter unter die Konnektionstüllen der Dialysemaschine verfahren werden, beziehungsweise rutschen. Der Aufnahmebereich 31 ist als rotationsbewegliches Element zu Beginn horizontal ausgerichtet, so dass der Konzentratbehälteradapter in diesem verbleibt. Nach Beendigung der Reinigung des Dialysatkreislaufs kann das rotationsbewegliche Element, der Aufnahmebereich 31, verkippen und damit den Konzentratbehälteradapter über das starre Element, den Gleitbereich 32, unter das Konnektorgehäuse 10 verfahren. Damit kann der Konzentratbeutel zu einer beliebigen Zeit, unabhängig von dem Reinigungsfortschritt, von dem Pflegepersonal, in die Aufnahmeeinrichtung 3 eingesetzt werden.

Fig. 12 zeigt eine Prinzipdarstellung einer Aufnahmeeinrichtung 3a nach einer zweiten Ausführungsform. Die Aufnahmeeinrichtung 3a weist einen Aufnahmebereich 31a mit Befestigungselement auf. In diesem Aufnahmebereich 31a kann der Konzentratbehälteradapter befestigt werden. Weiter weist die Aufnahmeeinrichtung einen Verfahrbereich 32a auf. Über diesen Verfahrbereich 32a kann der Abstand zwischen dem Aufnahmebereich 31a und dem Montagebereich verändert werden, so dass der Aufnahmebereich 31a mit dem aufgenommenen Konzentratbehälteradapter dem Konnektorgehäuse 10 zur Konnektion zugeführt werden kann. Zu Beginn legt das Pflegepersonal den Konzentratbehälteradapter in den Aufnahmebereich 31a ein. Dies kann unabhängig von dem Fortschritt der Reinigung geschehen. Nach Beendigung der Reinigung verfährt der Verfahrbereich 32a den Konzentratbehälteradapter zu dem Konnektorgehäuse 10, zur Konnektion unter die Konnektionstüllen. Der Verfahrbereich 32a kann als Teleskopmechanismus oder Klappmechanismus ausgebildet sein.

## Patentansprüche

1. Konzentratbehälteradapter für eine Blutbehandlungsvorrichtung, insbesondere eine Dialysemaschine, aufweisend
einen Befestigungskörper (1) an welchem ein Konzentratbeutel befestigbar ist, wobei der Befestigungskörper (1) eine Befüllöffnung (11) sowie zwei seitlich zur Befüllöffnung (1) angeordnete Verbindungsöffnungen (12a, 12b) aufweist,
einen Gehäuseaufnahmebereich (13) am Befestigungskörper (1),
wobei der Befestigungskörper (1) derart eingerichtet ist, dass mittels des Gehäuseaufnahmebereichs (13) der Konzentratbehälteradapter an einem Konnektorgehäuse (10) anbringbar ist,
und wobei der Gehäuseaufnahmebereich (13) außen an dem die Befüllöffnung (11) und/oder Verbindungsöffnungen (12a, 12b) bildenden Material angeordnet ist,
**dadurch gekennzeichnet, dass**
der Gehäuseaufnahmebereich (13) von der Befüllöffnung (11) und den Verbindungsöffnungen (12a, 12b) beabstandet ist.

2. Konzentratbehälteradapter nach Anspruch 1,
wobei der Gehäuseaufnahmebereich (13) einer Öffnungskontur der Befüllöffnung (11) folgt.

3. Konzentratbehälteradapter nach Anspruch 1,
wobei der Gehäuseaufnahmebereich (13) sich rahmenartig über zwei Längsstreben (14a, 14b) zu zwei gegenüberliegenden Seiten des Befestigungskörpers (1) von diesem wegerstreckt und zumindest eine Querstrebe (15b) aufweist, welche die Längsstreben (14a, 14b) verbindet.

4. Konzentratbehälteradapter nach Anspruch 3,
wobei die zumindest eine Querstrebe (15b) einen Vorsprung und/oder eine Einkerbung (17) aufweist, die derart eingerichtet ist, dass sie einen Bewegungsfreiheitsgrad des Konzentratbehälteradapters beschränkt, wenn dieser an einem Konnektorgehäuse (10) angebracht ist.

5. Konzentratbehälteradapter nach einem der vorhergehenden Ansprüche, weiter aufweisend
ein Konzentratbehälterrastelement (23a) am Befestigungskörper (1), das derart eingerichtet ist, dass es zumindest einen Bewegungsfreiheitsgrad des Konzentratbehälteradapters beschränkt, wenn dieses an einem Konnektorgehäuse (10) angebracht ist.

6. Konnektorgehäuse (10) zur Anbindung an eine Blutbehandlungsvorrichtung, insbesondere eine Dialysemaschine, aufweisend
zumindest einen Behälteraufnahmebereich (27a, 27b, 27c), der derart ausgebildet ist, dass er einen Konzentratbehälteradapter nach einem der Ansprüche 1 bis 5 an einem unteren Bereich des Konnektorgehäuses (10) aufnehmen kann, und
Konnektorverbindungselemente an einer Unterseite des Konnektorgehäuses (10), welche aus dem Konnektorgehäuse (10) verfahrbar sind, und derart eingerichtet sind, dass eine fluidische Verbindung mit dem Konzentratbehälteradapter herstellbar ist.

7. Konnektorgehäuse (10) nach Anspruch 6, wobei
der Behälteraufnahmebereich (27b) eine rillenförmige Aussparung auf der Oberseite des Konnektorgehäuses (10) ist.

8. Konnektorgehäuse (10) nach Anspruch 7, wobei
der rillenförmigen Aussparung ein Beschränkungselement zugeordnet ist, welches sich von und/oder zu der Oberseite des Konnektorgehäuses (10) wegerstreckt.

9. Konnektorgehäuse (10) nach einem der Ansprüche 6 bis 8, wobei
ein Konnektorgehäuserastelement an einer Unterseite des Konnektorgehäuses (10) ausgebildet ist.

10. Konnektorgehäuse (10) nach einem der Ansprüche 6 bis 9, weiter aufweisend
ein Türelement (24), mit einer ersten Haltevorrichtung (25) an einer Seite des Türelements (24),
eine zweite Haltevorrichtung zur beweglichen Befestigung des Türelements (24) an dem Konnektorgehäuse (10),
wobei der Behälteraufnahmebereich (27a) zur Aufnahme des Konzentratbehälteradapters auf dem Türelement (24) ausgebildet ist,
wobei das Türelement (24) derart um die zweite Haltevorrichtung verschwenkbar ist, so dass in einer ersten Position die der ersten Seite des Türelements (24) gegenüberliegende zweite Seite unter das Konnektorgehäuse (10) bewegbar ist, und in einer zweiten Position sich das Türelement (24) von dem Konnektorgehäuse (10) wegerstreckt.

11. Konnektorgehäuse (10) nach einem der Ansprüche 6 bis 10, wobei
ein Wandmaterial des Behälteraufnahmebereichs (27a) flach ausgebildet ist, oder der Behälteraufnahmebereich (27a) eine Kontur aufweist, welche zur Auflage des Konzentratbehälteradapters dient.

12. Konnektorgehäuse (10) nach Anspruch 10 oder 11, weiter aufweisend
ein erstes Eingriffelement (24a), das an der zweiten Seite des Türelements (24) ausgebildet ist,
ein zweites Eingriffelement (24b), das an dem Konnektorgehäuse (10) ausgebildet ist, so dass in der ersten Position des Türelements (24) das erste Eingriffelement (24a) mit dem zweiten Eingriffelement (24b) eingreifen kann.

13. Konnektorgehäuse (10) nach einem der Ansprüche 6 bis 12, weiter aufweisend
eine Erfassungseinheit zum Erfassen eines Konzentratbehälteradapters.

14. Konnektorgehäuse (10) nach Anspruch 13, wobei
die Erfassungseinheit ein Radar-Sensor zur Bewegungs- und/oder Lageerkennung des Konzentratbehälteradapters ist, und/oder die Erfassungseinheit zumindest ein Mikrotaster ist, und/oder die Erfassungseinheit ein Farbsensor und eine Lichtquelle ist, welche an der Unterseite des Konnektorgehäuses (10) angeordnet ist, und/oder die Erfassungseinheit eine Lichtsendeeinheit sowie eine Lichtempfangseinheit zum Erfassen eines Vorhandenseins einer Reflexionsgeometrie an einer bestimmten Position ist.

15. Konnektionssystem, aufweisend
ein Konnektorgehäuse nach einem der Ansprüche 6 bis 14, und
einen Konzentratbehälteradapter nach einem der Ansprüche 1 bis 5.

## Claims

1. Concentrate container adapter for a blood treatment device, in particular a dialysis machine, comprising
a fastening body (1) to which a concentrate bag can be fastened, the fastening body (1) having a filling opening (11) and two connecting openings (12a, 12b) arranged laterally to the filling opening (1),
a housing receiving region (13) on the fastening body (1),
the fastening body (1) being designed in such a way that the concentrate container adapter can be attached to a connector housing (10) by means of the housing receiving region (13),
and the housing receiving region (13) being arranged on the outside of the material forming the filling opening (11) and/or connecting openings (12a, 12b), **characterized in that**
the housing receiving region (13) is spaced apart from the filling opening (11) and the connecting openings (12a, 12b).

2. Concentrate container adapter according to Claim 1, wherein the housing receiving region (13) follows an opening contour of the filling opening (11).

3. Concentrate container adapter according to Claim 1, wherein the housing receiving region (13) extends away from the fastening body (1) in the manner of a frame via two longitudinal struts (14a, 14b) to two opposite sides of said fastening body and has at least one transverse strut (15b), which connects the longitudinal struts (14a, 14b) .

4. Concentrate container adapter according to Claim 3, wherein the at least one transverse strut (15b) has a projection and/or a notch (17), which is designed in such a way that it limits a degree of freedom of movement of the concentrate container adapter when the latter is attached to a connector housing (10).

5. Concentrate container adapter according to any one of the preceding claims, further comprising a concentrate container latching element (23a) on the fastening body (1), which latching element is designed in such a way that it limits at least one degree of freedom of movement of the concentrate container adapter when the latter is attached to a connector housing (10).

6. Connector housing (10) for attaching to a blood treatment device, in particular a dialysis machine, comprising
at least one container receiving region (27a, 27b, 27c), which is designed in such a way that it can receive a concentrate container adapter according to any one of Claims 1 to 5 on a lower region of the connector housing (10), and
connector connecting elements on a lower side of the connector housing (10), which connector connecting elements are movable out of the connector housing (10), and are designed in such a way that a fluidic connection with the concentrate container adapter can be established.

7. Connector housing (10) according to Claim 6, wherein the container receiving region (27b) is a groove-shaped recess on the upper side of the connector housing (10).

8. Connector housing (10) according to Claim 7, wherein the groove-shaped recess is assigned a limiting element which extends away from and/or to the upper side of the connector housing (10).

9. Connector housing (10) according to any one of Claims 6 to 8, wherein
a connector housing latching element is formed on a lower side of the connector housing (10).

10. Connector housing (10) according to any one of Claims 6 to 9, further comprising
a door element (24), with a first holding device (25) on one side of the door element (24),
a second holding device for movably fastening the door element (24) to the connector housing (10),
wherein the container receiving region (27a) for receiving the concentrate container adapter is formed on the door element (24),
the door element (24) being pivotable around the second holding device in such a way that, in a first position, the second side opposite the first side of the door element (24) is movable under the connector housing (10) and, in a second position, the door element (24) extends away from the connector housing (10).

11. Connector housing (10) according to any one of Claims 6 to 10, wherein
a wall material of the container receiving region (27a) is flat, or the container receiving region (27a) has a contour, which serves to support the concentrate container adapter.

12. Connector housing (10) according to Claim 10 or 11, further comprising
a first engagement element (24a), which is formed on the second side of the door element (24),
a second engagement element (24b), which is formed on the connector housing (10), such that, in the first position of the door element (24), the first engagement element (24a) can engage with the second engagement element (24b).

13. Connector housing (10) according to any one of Claims 6 to 12, further comprising
a detection unit for detecting a concentrate container adapter.

14. Connector housing (10) according to Claim 13, wherein
the detection unit is a radar sensor for the motion and/or position detection of the concentrate container adapter, and/or the detection unit is at least one micro-pushbutton, and/or the detection unit is a colour sensor and a light source, which is arranged on the lower side of the connector housing (10), and/or the detection unit is a light-transmitting unit and a light-receiving unit for detecting the presence of a reflection geometry at a certain position.

15. Connection system, comprising
a connector housing according to any one of Claims 6 to 14, and
a concentrate container adapter according to any one of Claims 1 to 5.

## Revendications

1. Adaptateur de conteneur de concentré pour un dispositif de traitement de sang, en particulier une machine de dialyse, comprenant
un corps de fixation (1) sur lequel est apte à être fixée une poche de concentré, le corps de fixation (1) présentant une ouverture de remplissage (11) ainsi que deux ouvertures de raccordement (12a, 12b) agencées latéralement par rapport à l'ouverture de remplissage (1),
une zone (13) de réception de boîtier sur le corps de fixation (1),
le corps de fixation (1) étant conçu de telle sorte que l'adaptateur de conteneur de concentré soit apte à être fixé à un boîtier de connecteur (10) au moyen de la zone (13) de réception de boîtier,
et la zone (13) de réception de boîtier étant agencée à l'extérieur du matériau formant l'ouverture de remplissage (11) et/ou les ouvertures de raccordement (12a, 12b),
**caractérisé en ce que**
la zone (13) de réception de boîtier est espacée de l'ouverture de remplissage (11) et des ouvertures de raccordement (12a, 12b).

2. Adaptateur de conteneur de concentré selon la revendication 1,
dans lequel la zone (13) de réception de boîtier suit le contour de l'ouverture de remplissage (11).

3. Adaptateur de conteneur de concentré selon la revendication 1,
dans lequel la zone (13) de réception de boîtier s'étend à l'écart de celui-ci à la manière d'un cadre, par deux entretoises longitudinales (14a, 14b), vers deux côtés opposés du corps de fixation (1), et présente au moins une entretoise transversale (15b) qui relie les entretoises longitudinales (14a, 14b).

4. Adaptateur de conteneur de concentré selon la revendication 3,
dans lequel ladite au moins une traverse (15b) présente une saillie et/ou une encoche (17) qui est conçue de telle sorte qu'elle limite le degré de liberté de mouvement de l'adaptateur de conteneur de concentré lorsque celui-ci est fixé à un boîtier de connecteur (10).

5. Adaptateur de conteneur de concentré selon l'une des revendications précédentes, comprenant en outre
un élément (23a) de verrouillage de conteneur de concentré sur le corps de fixation (1), qui est conçu de telle sorte qu'il limite au moins un degré de liberté de mouvement de l'adaptateur de conteneur de concentré lorsque celui-ci est fixé à un boîtier de connecteur (10).

6. Boîtier de connecteur (10) pour le raccordement à un dispositif de traitement de sang, en particulier une machine de dialyse, comprenant
au moins une zone (27a, 27b, 27c) de réception de conteneur, qui est conçue de telle sorte qu'elle soit apte à recevoir un adaptateur de conteneur de concentré selon une des revendications 1 à 5 dans une zone inférieure du boîtier de connecteur (10), et
des éléments de raccordement de connecteur sur une face inférieure du boîtier de connecteur (10), qui sont aptes à être déplacés hors du boîtier de connecteur (10) et sont conçus de telle sorte qu'une liaison fluidique avec l'adaptateur de conteneur de concentré est apte à être établie.

7. Boîtier de connecteur (10) selon la revendication 6, dans lequel
la zone (27b) de réception de conteneur est un évidement en forme de rainure sur la face supérieure du boîtier de connecteur (10).

8. Boîtier de connecteur (10) selon la revendication 7, dans lequel
l'évidement en forme de rainure est associé à un élément de restriction qui s'étend depuis, et/ou vers, la face supérieure du boîtier de connecteur (10).

9. Boîtier de connecteur (10) selon l'une des revendications 6 à 8, dans lequel
un élément de verrouillage de boîtier de connecteur est formé sur une face inférieure du boîtier de connecteur (10).

10. Boîtier de connecteur (10) selon l'une des revendications 6 à 9, dans lequel
un élément (24) formant porte, avec un premier dispositif de maintien (25) sur un côté de l'élément (24) formant porte,
un deuxième dispositif de maintien pour fixer de manière mobile l'élément (24) formant porte sur le boîtier de connecteur (10),
la zone (27a) de réception de conteneur étant conçue de façon à recevoir l'adaptateur de conteneur de concentré sur l'élément (24) formant porte,
l'élément (24) formant porte étant apte à pivoter autour du deuxième dispositif de maintien de telle sorte que, dans une première position, le deuxième côté opposé au premier côté de l'élément (24) formant porte soit apte à être déplacé sous le boîtier de connecteur (10), et que, dans une deuxième position, l'élément (24) formant porte s'étende à l'écart du boîtier de connecteur (10).

11. Boîtier de connecteur (10) selon l'une des revendications 6 à 10, dans lequel
un matériau de paroi de la zone (27a) de réception de conteneur est plat, ou la zone (27a) de réception de conteneur présente un contour qui sert à soutenir l'adaptateur du conteneur de concentré.

12. Boîtier de connecteur (10) selon la revendication 10 ou la revendication 11, comprenant en outre
un premier élément d'engagement (24a), formé sur le deuxième côté de l'élément (24) formant porte,
un deuxième élément d'engagement (24b), formé sur le boîtier de connecteur (10), de sorte que, dans la première position de l'élément (24) formant porte, le premier élément d'engagement (24a) soit apte à venir en engagement avec le deuxième élément d'engagement (24b).

13. Boîtier de connecteur (10) selon l'une des revendications 6 à 12, comprenant en outre
une unité de détection, pour détecter un adaptateur de conteneur de concentré.

14. Boîtier de connecteur (10) selon la revendication 13, dans lequel
l'unité de détection est un capteur radar pour la détection du mouvement et/ou de la position de l'adaptateur du conteneur de concentré, et/ou l'unité de détection est au moins un micro-capteur, et/ou l'unité de détection est un capteur de couleur et une source de lumière, qui est agencée sur la face inférieure du boîtier de connecteur (10), et/ou l'unité de détection est une unité émettrice de lumière ainsi qu'une unité réceptrice de lumière pour détecter la présence d'une géométrie de réflexion en une position déterminée.

15. Système de connexion, comprenant
un boîtier de connecteur selon l'une des revendications 6 à 14, et
un adaptateur de conteneur de concentré selon l'une des revendications 1 à 5.
